# EUROPEAN PATENT SPECIFICATION

(11) **EP 3 225 655 B1**
(45) Date of publication and mention of the grant of the patent: **12.12.2018**
(21) Application number: 15813241.5
(22) Date of filing: 29.10.2015
(51) Int. Cl.: C08J 11/16, C08J 11/18, C08J 11/28, C22B 1/20, C07C 51/09

(54) **PROCESS FOR OBTAINING ORE DUST SUPPRESSANT RESIN, ORES DUST SUPPRESSANT RESIN, PROCESS FOR INHIBITION OF ORE PARTICULATE EMISSION AND RESIN USE**
VERFAHREN ZUR GEWINNUNG VON ERZSTAUBBINDEMITTELHARZ, ERZSTAUBBINDEMITTELHARZ, VERFAHREN ZUR HEMMUNG VON ERZPARTIKELEMISSION UND HARZVERWENDUNG
PROCÉDÉ D'OBTENTION D'UNE RÉSINE ANTI-POUSSIÈRE DE MINERAI, RÉSINE ANTI-POUSSIÈRE DE MINERAIS, PROCÉDÉ D'INHIBITION DE L'ÉMISSION DE MATIÈRES PARTICULAIRES DE MINERAIS ET UTILISATION DE CETTE RÉSINE

(30) Priority: 28.11.2014 BR 102014029870
(43) Date of publication of application: 04.10.2017
(73) Proprietor: Vale S.A., 22250-145 Rio de Janeiro, RJ (BR); Universidade Federal Do Espírito Santo - Ufes, Vitória - ES CEP-29075-910 (BR)
(72) Inventor: VASCONCELOS, Renata Eliane Frank, 29055-460 Vitória - ES (BR); DA SILVA FILHO, Eloi Alves, 29065-440 Vitória - ES (BR); DE MELO, Carlos Vital Paixã, 29065-450 Vitória - ES (BR)
(74) Representative: EP&C
(86) International application number: PCT/BR2015/000165
(87) International publication number: WO 2016/082010

(56) References cited:
- BR-A2-102013 001 662
- JP-A- 2005 002 403
- US-A- 5 958 987
- US-A1- 2013 261 197

## Description

### FIELD OF THE INVENTION AND SHORT DESCRIPTION

The present invention relates to the technical mining and recycling of polymer materials. More specifically, the invention relates to a process for obtaining a resin used as ore dust suppressant. The process derives from the chemical recycling of the polyethylene terephthalate thermoplastic polymer (or PET). A method for obtaining the ore dust suppressant resin is disclosed by using the depolymerization reaction methodology of polyethylene terephthalate polymer obtained from post-consumption PET bottles (PETpc) in the presence of a cationic surfactant, hexadecyltrimethylammonium bromide (CTAB). A process for inhibiting the emission of ore particulate by the use of ore dust suppressant resin as obtained in the present invention is also part of the invention.

### STATE OF THE ART

The mining industry is of great importance for the economy of many countries. According to common knowledge from the state of the art the ores are marketed (especially iron ore) in its natural form or compressed, for example in the form of pellets. In the second case, before being transported, these pellets are subjected to heat treatment in the factory furnaces and then are handled, packaged and transported. Although this procedure is regularly used it is of common knowledge that there are many disadvantages arising from this production process, including the formation of large amounts of fine particles or ore dust or ore particulate due to the continuous friction between the pellets.

This ore dust is released into the environment and ends up reaching the facilities of the ports and the communities that are close to ports and factories in the cities where mining and pelletizing processes of ores have an important economic role. The emission of these particles cause in addition to health problems, disorders of everyday life of the residents of the surrounding communities and environmental problems.

Various particulate emission inhibitors have already been described by the state of the art, such as water, polymers, mineral oils and alcohol derivatives, however, the development of dust suppressants that are efficient, environmentally friendly and economically viable remains a current challenge.

The polyethylene terephthalate is a thermoplastic polymer member of a class of polymers containing ester functional group in its main chain, known as polyesters. It is widely used in the manufacture of plastic packages, primarily for beverages, due to its high transparency and resistance. In recent years the demand for these plastics has increased significantly, which results in two major potential problems in PET production chain. The first of these problems relates to the origin of the raw material for the production of PET, as like all polymers, polyesters are made of derivatives of refinement and petroleum reforming material (petrochemical raw materials) and the raw material is impacted by high costs and the fact that its source is not renewable. The second problem is environmental and refers to the disposal of products made from PET, especially plastic bottles and other plastic utensils that become serious pollution agents as they are produced in huge quantities and discarded. The disposal of plastic waste is a worldwide chronic problem. Thus, economic, environmental and sustainability motivations mobilize the search for improvements and innovations related to PET recycling and the utilization of its sub products. The present invention surprisingly discloses that it is possible to obtain an ore dust suppressant resin by using sub products of the recycling of plastics, particularly post-consumption PET (PETpc).

Some plastic recycling processes or polymeric materials are known from the state of the art, that seek to both reuse these materials and their decomposition in order to obtain recycled raw materials that can return to the productive chain of manufacturing polymers or be used for other purposes with commercial interests.

For each type of polymeric material there are specific recycling methods. In general, the separation of plastics starts the recycling process and must be made considering the physical properties of polymers such as density, thermal conductivity, softening temperature, among other properties.

The classification of plastic is made depending on the types of changes needed for recycling, which are defined in four types: **primary or pre-consumption,** aimed at the reuse of industrial polymer waste and getting products with characteristics similar to the original product; **secondary or post-consumption,** aimed at the transformation of polymeric waste from municipal solid waste and getting products that have less demanding than virgin polymer and are used in the production of other materials; **tertiary,** also known as **chemical recycling** involving the production of fuels or chemical products from polymeric and where post-consumer waste plastics are processed into monomers and reused in production of new plastics with quality similar to the original polymer; and, finally, **quaternary recycling,** also called **energy recycling,** where energy recovery of polymer waste occurs by controlled incineration. In these types of recycling also exists the **mechanical recycling,** which is performed through the reprocessing of plastics by extrusion, obtaining polymeric material transformed into pellets (plastic grains).

In the case of post-consumption PET type plastics, the main transformation type currently used for decomposition is the tertiary or chemical recycling, also known as chemical depolymerisation or decomposition. The PET chemical decomposition is based on the reversible polymerization reaction and can be performed by chemical processes of hydrolysis, glycolysis, methanolysis and aminolysis, and it can be catalyzed by acids, bases or neutral catalysts.

For hydrolysis PET is depolymerized into its monomers, terephthalic acid and ethylene glycol. After purification these materials can be used for polymerization or for other purposes of commercial importance, providing economy and reducing the demand for petroleum products, non-renewable raw materials.

Some studies in the bibliography use only sodium hydroxide solution and methanol in the depolymerization reaction, and other studies use zinc acetate as a catalyst, but in general the processes are characterized by an average time reaction during from 3 to 6 hours, which is a high energy cost.

The present invention discloses the use of cationic surfactants, preferably hexadecyltrimethylammonium bromide (CTAB), in the process of obtaining dust suppressant resin from the chemical decomposition of PET demonstrated surprising results in relation to the characteristics of the process and of the final product.

The review article by Daniel Passion and Tadeusz Spychaj [Ind. Eng. Chem. Res. 1997, vol.36, p.1373-1383, 1997] presents the advances for recycling of PET started in the 80s. These developments were important for the reduction of process costs, however, other catalysts are also mentioned but no process used surfactants as catalyst.

SOUZA et.al., seeking to optimize the PET depolymerization process made use of the anionic surfactant sodium dodecyl sulfate (DDS) and non-ionic surfactants Tween™ type (polyethoxylated sorbitan esters derived from fatty acids), seeking to increase efficiency in PET chemical recycling, by hydrolysis in basic medium in order to obtain the terephthalic acid for purposes of repolymerization [SOUZA, L.; TORRES, M.C.M.; RUVOLO SON, A.C. Depolymerization of Poly (Ethylene Terephthalate) - PET: Efeitos de Tensoativos e Excesso de Solução Alcalina. Polymers: Ciência e Tecnologia, vol.18, No. 4, p.334-341, 2008]. The result observed was that the use of surfactants resulted in increase in process efficiency, but also impurities added to the recovered terephthalic acid.

The processes presented so far to the depolymerization of PET provided the drawback of requiring long periods of reaction and high energy consumption to obtain as end-product the terephthalic acid and ethylene glycol, which ended up demanding a significant cost in the recycling chain.

The Brazilian patent PI0200325**,** for example, describes the obtaining of terephthalic acid by means of chemical recycling of PET made from a polymer reaction with sodium hydroxide under a maximum pressure of 15 atm and temperatures up to 198°C obtained with a heating rate between 15 and 25°C/min. At the end of the reaction, the reactor is cooled and disodium terephthalate, solid reaction product is separated by filtration. The disodium terephthalate solution is brought to react with sulfuric acid until the solution pH reaches the value 3 when all the terephthalic acid precipitates, necessitating its filtering, drying, grinding and screening.

Based on the knowledge of the work initiated by SOUZA et.al., the invention described in the patent request BR102013001662-4 describes the depolymerisation reaction of post-consumption PET bottle using the cationic surfactant hexadecyltrimethylammonium bromide (CTAB) in alkaline hydrolysis, in order to obtain the terephthalic acid monomer.

The present invention discloses a process that, through the use of catalysts and specific reaction conditions, allows the chemical decomposition of PET to be carried out in a rapid, efficient methodology and of low cost for obtaining an intermediate resin containing ethylene glycol being that from this intermediate resin is then possible to obtain the interest powder suppressant resin.

Surprisingly, in this invention, the chemical decomposition of the PET is performed at temperatures lower than those described in the state of the art, and there is the need for control of pressure and heating rate. Furthermore, the process of the present invention uses a simple reaction system which needs only a backflow connector to cool the process during the reaction and prevent losses by volatilization of the reactants. Another interesting factor in the present invention is the use of CTAB surfactant as a catalyst and reduction in depolymerisation reaction time.

The relevance of the present invention can also be highlighted by the fact that a ton of PET decomposed generates on average 300 liters of ethylene glycol.

### OBJECTIVES AND ADVANTAGES OF THE INVENTION, THE PROPOSED SOLUTION FOR THE EXISTING PROBLEM, ADVANTAGES IN RELATION TO THE STATE OF THE ART.

The invention relates to a process for obtaining mineral powder suppressant resin by means of chemical recycling of PET thermoplastic polymer. The process of the present invention uses the depolymerization reaction methodology of PET polymer obtained primarily from post-consumption PET bottle waste, in the presence of a cationic surfactant, preferably hexadecyltrimethylammonium bromide (CTAB).

One of the objectives of the present invention is the sustainable recovery and recycling of polymer materials in order to obtain a mineral powder suppressant resin.

The process of the present invention consists in producing an intermediate resin containing ethylene glycol which is obtained as a sub product of the chemical recycling process of post-consumption PET, such resin being used for the subsequent obtaining of a mineral powder suppressant, that is with the aim of solving the drawback caused by the generation of powder in wagons and ore stacks.

However, the depolymerisation process of the present invention which occurs in the presence of cationic surfactant as a catalyst, in addition to the technical advantages presented, also provides for the generation of terephthalic acid as a sub product of the decomposition of PET, and it is noteworthy that the terephthalic acid has significant commercial value aggregate after its extraction.

Another advantage of the invention is the obtaining a commercially viable product as a result of a recycling process which represents a cost effective alternative disposal for companies and communities waste. It is also worth mentioning that in the process of the proposed invention, the PET can be in the form of granules of virgin material, industrial waste flakes or post-consumption, as well as in various particle sizes and colors. PET origin color is also irrelevant and it can be used, for example, PET bottles of either green or transparent color, without loss to the reaction time and the quality or purity of the final product. This also contributes to the fast process, because additional steps for PET separation are not necessary according to their color of origin.

In addition the present invention allows the aggregation of the commercial value of the recycling product since the market values of the ore powder suppressant resulting from the process, or even the intermediates terephthalic acid and ethylene glycol, are much higher than the market value of the original raw material (gross mass of polymer, particularly PET, originated from selection and recycling processes without additional processing).

Another advantage of the present invention is its contribution to environmental development and sustainability, as its process uses as raw material plastic waste and that the use of this process also allows the qualification of waste pickers and their families (usually unqualified work) to obtain the raw material by offering them basic working conditions and income generation.

Also another advantage of the invention is that through the use of the process object of the invention it is possible to promote the development of the surrounding communities of large companies and producing communities of large amounts of polymer waste as disposal, in addition to benefit communities and collectors cooperatives involved in selective collection and recycling chain, through the sale of products derived from recycling and dust suppressant.

One more advantage of the present invention process is the fact that it is a process of simple execution, enabling be used by any company that uses ore stacks yards and/or transport its ore by rail. Especially projects installed in remote areas, distant from industrial centers, where logistical costs represent an obstacle for removing polymeric residues may benefit from this invention.

### BRIEF DESCRIPTION OF THE FIGURES

Figure 1 is a representation of chemical reactions of PETpc depolymerization occurring in the process object of the present invention, shown without the addition of CTAB (Reaction I) and with the addition of CTAB (Reaction II).
Figure 2 illustrates the characterization of the PET resin by infrared spectroscopy in transmission mode.
Figure 3 illustrates the thermogravimetric analysis (TGA) of the product, and the experiments conducted at a heating rate of 10°C·min⁻¹, in an inert atmosphere of N₂ and oxidant (synthetic air) at a temperature range of 30 to 450°C. Figure 3 (a) refers to TPA and figure 3 (b) to PETpc.
Figure 4 shows the DSC curves for samples of (a) PETpc and (b) TPA.

### DETAILED DESCRIPTION OF THE INVENTION

For performing the present invention it is common that the post-consumption PET plastic (PETpc) is subjected to a preliminary process of recycling and cleaning before depolymerization reaction comprising i) selecting waste plastic composed of PETpc from selective collect; ii) removing portions of different materials to PETpc from waste plastics (for example, bottle top and bottom); iii) washing; iv) drying; v) grinding and standardization of fragment size.

After the cleaning process it is initiated the PETpc chemical recycling process itself, comprising the following steps: i) the depolymerization PETpc clean fragments in the presence of a cationic surfactant and an alkaline medium; ii) neutralization of the reaction medium after completion of the depolymerization reaction and precipitation of the terephthalic acid monomer (TPA); iii) filtration of the remaining medium containing ethylene glycol and extraction of complementary salt excess with alcoholic solvent; iv) subjection of the solution obtained in step iii) to an additional evaporation process to remove excess water, thus obtaining an intermediate resin; v) addition of a viscosity increasing agent to the intermediate resin obtained in step iv) obtaining a ore dust suppressant resin, and optionally vi) addition of an agent to increase the hydrophobicity of the ore dust suppressant resin obtained in step v).

The cationic surfactant used in step i) is hexadecyltrimethylammonium bromide (CTAB).

The depolymerization reaction as described in step i) is carried out for 1 to 2 hours, while the temperature is maintained within a range of 90 to 110°C.

In the extraction of excess complement salt with alcoholic solvent of step iii) the extraction alcoholic solvent used can be any alcoholic solvent, being preferably selected from the group consisting of isopropyl alcohol, ethanol, and methanol, and being preferably the isopropyl alcohol. After its use, the alcohol solvent can be recovered by distillation and reused in another stage salt removal. There is no discarding of the alcohol solvent, and it can be reused more times, until it is completely consumed.

The viscosity increasing agent added in step v) is selected from the group consisting of pyrrolidones, being preferably polyvinylpyrrolidone.

The agent for increasing the hydrophobicity optionally added in step vi) is selected from the group consisting of lignin obtained from vegetable and polyethylene wax, preferably with the lignin obtained from leaves and branches of trees through extraction with 50% ethanol-water mixture. The more hydrophobic is the resin, more efficient it is for use in ores.

The resin product was characterized, in structural terms, as described below.

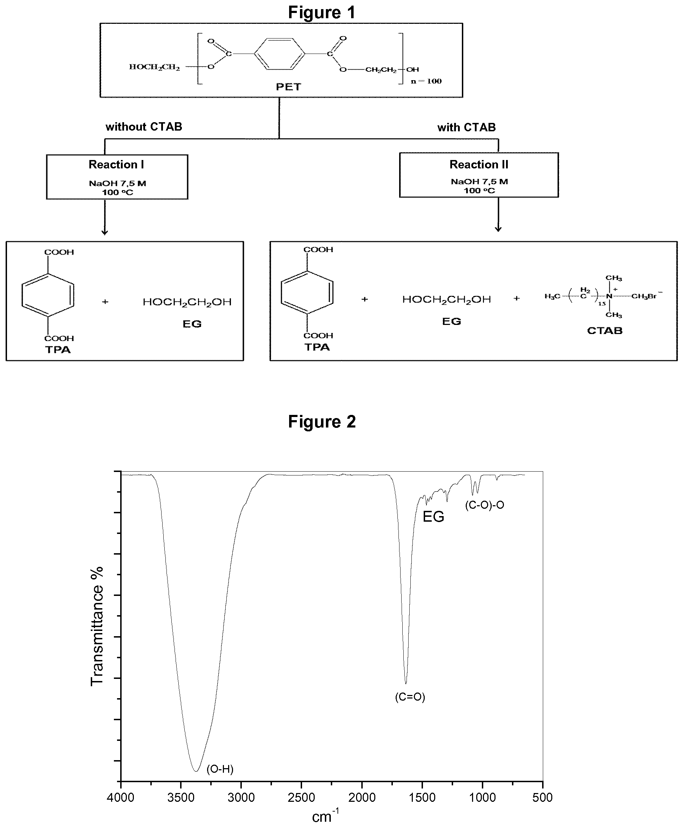

The characterization of PET resin by infrared spectroscopy was performed in a FTIR, FTLA 2000-102 (ABBBOMEM) spectrometer, in transmission mode. The analyzes were recorded with a resolution of 4 cm⁻¹ in a wavelength range of 4000 of the 500 cm⁻¹ and an average of 32 scans. The spectrum obtained is shown in Figure 2, where it was observed that the resin comprises characteristic functional groups on the five absorption peaks 3373, 1457, 1296, 1075 and 1037 cm⁻¹ respectively.

The assignments of peaks are for axial deformation in the region of 3373 cm⁻¹ for the O-H group; 1639 cm⁻¹ for the C=O group; 1457 and 1296 and the ethylene glycol (EG) and 1075 for the (C=O)-O group, where differences are observed in the appearance of intense and broad absorption in the region of 3373 cm⁻¹. This spectrum shows the specificity of PET resin, where it was possible to observe the PET depolymerization products.

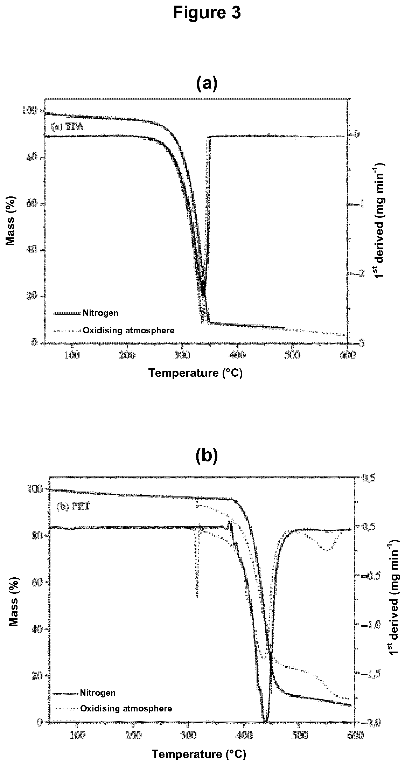

Thermogravimetric analysis (TGA) was performed on a Shimadzu TG-50 equipment, where 10 mg of sample were used for analysis, and the experiments were conducted at a heating rate of 10°C·min⁻¹, in an inert atmosphere of N₂ and oxidizing (synthetic air) at a temperature range of 30 to 450°C, shown in Figure 3.

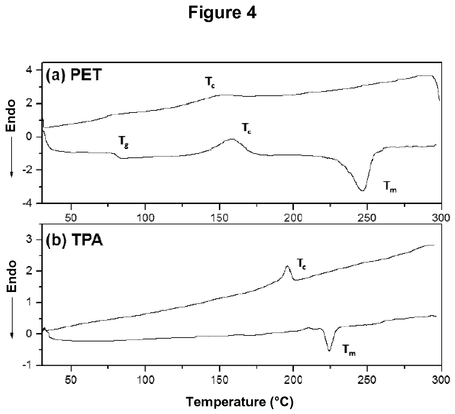

The Differential Scanning Calorimetry Analysis (DSC) was performed on a Q100 equipment (TA Instruments) controlled by Universal V4.7 software (TA Instruments). The data were obtained at heating and cooling rates of 10°C per minute, with N₂ flow of 50 mL·min⁻¹ in the temperature range of 25 to 260°C, shown in Figure 4.

These results show that Figure 3 (a,b) has a thermal decomposition range in the range of 250 to 350°C. However, the PETpc had greater thermal stability in the range of 310 to 600°C in oxidizing atmosphere and of 370 to 500°C in an inert atmosphere. The first mass loss is due to the presence of comonomers such as diethylene glycol (DEG). The second mass loss is the presence of EG in the carbon chain of PETpc.

The results of DSC Calorimetry, Figure 4 represents the cooling and heating curves for PETpc and TPA respectively.

The resin obtained was originated from PET pc and thus on its composition produced TPA after depolymerization reaction, where the cooling curve does not show a defined crystallization peak (Tc), so the material has a slow crystallization kinetic, justifying its high molecular mass and the presence of copolymers that retard the crystallization process (the property that provides the desired transparency to PET during processing by injection-blow), Figure 4(a). The crystallization of PETpc is only completed when the second heating curve is performed, and it is observed a T_{c} = 158°C, Figure 4(a). The second heating curve showed a glass transition temperature, T_{g} = 81°C and the melting temperature, Tm = 247°C for PET_{pc} in accordance with the work published by Prof. group De Paoli (Spinace, M.A.; De Paoli, M-A., J. Appl. Polym. Sci, 78, p.20 (2001)). On the other hand, in Figure 4(b), there was a peak of Tc = 190°C and Tm = 225°C which are typical for samples of TPA recovered from depolymerization of PETpc and present products of the resin preparation.

Pour point of he resin was also determined, by the manual method, in which the determined value of the pour point for the PET-UFES resin was - 22°C, and this shows that the PET resin shows a good stability in critical conditions of low temperatures.

In a preferred embodiment, the depolymerization reaction described in step i) of the process of the present invention is carried out in an alkaline medium (NaOH 7.5 mol/L) at a temperature of 100°C in a stainless steel reactor under temperature, pressure, time and pH control.

The following examples are presented for better understanding of preferred embodiments of the present invention, which are not limiting the scope thereof.

**Example** 1: PETpc depolymerization reaction in the presence of CTAB surfactant. The depolymerization reaction is carried out using PETpc previously cleaned with water and detergent and dry, then ground into a size of 1cm X 1cm. The PETpc fragments are added to a flat bottom flask with three joints with a capacity of 1000 mL, in the presence of 650 mL of sodium hydroxide solution (NaOH) at a concentration of 7.5 mol/L in the presence of 160 mL of cationic surfactant CTAB, kept under constant stirring for 60 minutes at 100°C

After 60 min of the depolymerization reaction it was added concentrated hydrochloric acid to neutralize NaOH and precipitation of the monomer terephthalic acid (TPA) with sodium chloride salt (NaCI), which are removed by filtration. The remaining medium containing ethylene glycol, is filtered under vacuum and isopropyl alcohol is added to remove excess sodium chloride salt (NaCI). The solution obtained is again subjected to an evaporation process at 100°C for removal of excess water, obtaining 200 mL of the intermediate resin.

### Example 2: preparation of dust suppressant resin.

At 200 mL of the intermediate resin obtained as described in Example 2 it was added 10g of the product PVP K-90 (polyvinylpyrrolidone) in order to increase the final viscosity, obtaining the ore dust suppressant resin with a density of d=1.17g/mL and viscosity of η=55.6 mm²/s or 55.6 cSt.

The results presented in Examples 1 and 2 demonstrate the relevance and inventiveness of the present invention when demonstrating that the presence of the cationic surfactant in the reaction medium allows the reaction to obtain the intermediate resin is carried out at a much lower time (2h according to Example 1) compared to the reaction time of the reaction without the presence of cationic surfactant (average time of 6 hours, according to the state of the art). Besides the significant reduction in reaction time it is emphasized that high purity products are obtained by the process of the present invention. Figure 1 shows the schemes of the chemical reactions of PETpc depolymerization, with or without the use of CTAB surfactant as catalyst.

Alternatively, also as part of the invention, to the ore dust suppressant resin may be added other components to make the resin more hydrophobic. As example of an additive, but without limiting the understanding of the scope of the present invention, plant lignin can be added (such as leaves and tree branches) obtained by extraction with a mixture of 50% ethyl alcohol and 50% distilled water.

Having the present invention been described in the form of its preferred embodiments and examples, it should be understood that other possible variations are covered by the scope of the present invention, which is limited only by the contents of their claims, including possible equivalent modifications.

## Claims

1. ORE DUST SUPPRESSANT RESIN, **characterized by** the fact that it is a resin of depolymerization ethylene terephthalate polymer product obtained by a process comprising the steps of:
i) depolymerization of clean fragments of post-consumer polyethylene terephthalate (PETpc) in the presence of hexadecyltrimethylammonium bromide (CTAB) and an alkaline medium for a duration of 1 to 2 hours, at a temperature in the range of 90 to 110°C;
ii) neutralization of the reaction medium after completion of the depolymerization reaction and precipitation of the terephthalic acid monomer (TPA);
iii) filtration of the remaining medium containing ethylene glycol and extraction of excess of complementary salt with alcoholic solvent;
iv) subjection of the solution obtained in step iii) to an evaporation process to remove excess water, thus obtaining an intermediate resin;
v) addition of an viscosity increasing agent selected from the group consisting of pyrrolidones, preferably polyvinylpyrrolidone, to the intermediate resin obtained in step iv), obtaining an ore dust suppressant resin, and optionally
vi) addition of an agent to increase the hydrophobicity of the ore dust suppressant resin obtained in step v).

2. RESIN, according to claim 1, **characterized by** the fact of being composed of characteristic functional groups in five absorption peaks in the infrared region (IV) of 3373, 1457, 1296, 1075 and 1037 cm⁻¹ respectively.

3. PROCESS FOR INHIBITION OF ORE PARTICULATE EMISSIONS, **characterized by** the fact that it uses the ore dust suppressant resin as defined in one of the claims 1 to 2.

4. USE OF THE RESIN, as defined in one of the claims 1 to 2, **characterized by** the fact that it is for ore dust suppression.

## Patentansprüche

1. Erzstaubbindemittelharz, **gekennzeichnet durch** die Tatsache, dass es ein Harz eines Depolymerisationsproduktes von Ethylenterephthalatpolymer ist, das durch ein Verfahren erhalten wird, das die Schritte umfasst:
i) Depolymerisation von sauberen Fragmenten von Post-Consumer-Polyethylenterephthalat (PETpc) in Gegenwart von Hexadecyltrimethylammoniumbromid (CTAB) und einem alkalischen Medium für eine Dauer von 1 bis 2 Stunden bei einer Temperatur im Bereich von 90 bis 110°C;
ii) Neutralisation des Reaktionsmediums nach Abschluss der Depolymerisationsreaktion und Fällung des Terephthalsäuremonomers (TPA);
iii) Filtration des restlichen Mediums, das Ethylenglykol enthält, und Extraktion von überschüssigem Komplementsalz mit alkoholischem Lösungsmittel;
iv) Unterwerfen der in Schritt iii) erhaltenen Lösung einem Verdampfungsverfahren, um überschüssiges Wasser zu entfernen, um so ein Zwischenharz zu erhalten;
v) Zugabe eines viskositätserhöhenden Mittels, ausgewählt aus der Gruppe, bestehend aus Pyrrolidonen, vorzugsweise Polyvinylpyrrolidon, zu dem in Schritt iv) erhaltenen Zwischenharz, um ein Erzstaubbindemittelharz zu erhalten, und gegebenenfalls
vi) Zugabe eines Mittels zur Erhöhung der Hydrophobie des in Schritt v) erhaltenen Erzstaubbindemittelharzes.

2. Harz nach Anspruch 1, **gekennzeichnet durch** die Tatsache, dass es aus charakteristischen funktionellen Gruppen in fünf Absorptionspeaks im Infrarotbereich (IV) von 3373, 1457, 1296, 1075 bzw. 1037 cm⁻¹ besteht.

3. Verfahren zur Hemmung von Erzteilchenemissionen, **gekennzeichnet durch** die Tatsache, dass es das Erzstaubbindemittelharz, wie in einem der Ansprüche 1 bis 2 definiert, verwendet.

4. Verwendung des Harzes, wie in einem der Ansprüche 1 bis 2 definiert, **gekennzeichnet durch** die Tatsache, dass es zum Binden von Erzstaub dient.

## Revendications

1. RESINE ABAT-POUSSIERE DE MINERAI, **caractérisée par le fait qu'**il s'agit d'un produit de dépolymérisation du polymère poly(téréphtalate d'éthylène) obtenu par un procédé comprenant les étapes de:
i) dépolymérisation de fragments propres de polyéthylène téréphtalate (PETpc) après consommation en présence de bromure d'hexadécyltriméthylammonium (CTAB) et d'un milieu alcalin pendant 1 à 2 heures à une température maintenue dans la plage allant de 90°C à 110°C;
ii) neutralisation du milieu de réaction après achèvement de la réaction de dépolymérisation et précipitation du monomère acide téréphtalique (TPA);
iii) filtration du milieu restant contenant de l'éthylène glycol et extraction d'excès de sel complémentaire avec un solvant alcoolique;
iv) soumission de la solution obtenue à l'étape iii) à un procédé d'évaporation pour éliminer de l'eau en excès, obtenant ainsi une résine intermédiaire;
v) addition d'un agent augmentant la viscosité choisi dans le groupe constitué par les pyrrolidones, de préférence de la polyvinylpyrrolidone, à la résine intermédiaire obtenue à l'étape iv), obtention d'une résine abat-poussière de minerai et éventuellement
vi) addition d'un agent pour augmenter l'hydrophobicité de la résine abat-poussière de minerai obtenue à l'étape v).

2. RESINE selon la revendication 1, **caractérisée par le fait qu'**elle est composée de groupes fonctionnels caractéristiques présentant cinq pics d'absorption dans la région infrarouge (IV) de 3373, 1457, 1296, 1075 et 1037 cm⁻¹ respectivement.

3. PROCEDE D'INHIBITION D'EMISSIONS DE PARTICULES DE MINERAI, **caractérisé par** le fait d'utiliser la résine abat-poussière de minerai telle que définie dans l'une des revendications 1 à 2.

4. UTILISATION DE RESINE définie dans l'une des revendications 1 à 2, **caractérisée par le fait qu'**elle est destinée à la suppression de poussière de minerai.
